# EUROPÄISCHE PATENTSCHRIFT

(11) **EP 4 551 169 B1**
(45) Veröffentlichungstag und Bekanntmachung des Hinweises auf die Patenterteilung: **01.10.2025**
(21) Anmeldenummer: 24762618.7
(22) Anmeldetag: 27.08.2024
(51) Int. Cl.: A61F 5/11

(54) **NAGELSPANGENAPPLIKATOR ZUR NAGELSPANGENAPPLIKATION**
NAIL CLASP APPLICATOR FOR NAIL CLASP APPLICATION
APPLICATEUR D'ONGLES POUR L'APPLICATION DE L'ONGLE

(30) Priorität: 21.09.2023 DE 102023209249; 27.09.2023 DE 202023105639 U
(43) Veröffentlichungstag der Anmeldung: 14.05.2025
(73) Patentinhaber: Stolz, Bernd, 92224 Amberg (DE)
(72) Erfinder: Stolz, Bernd, 92224 Amberg (DE)
(74) Vertreter: Rau, Schneck & Hübner Patentanwälte Rechtsanwälte PartGmbB
(86) Internationale Anmeldenummer: PCT/EP2024/073893
(87) Internationale Veröffentlichungsnummer: WO 2025/061421

(56) Entgegenhaltungen:
- EP-A1- 2 596 772
- CN-U- 208 808 794
- JP-A- 2013 111 477
- KR-U- 20140 000 398

## Beschreibung

Die Erfindung betrifft einen Nagelspangenapplikator und ein Verfahren zur Applikation einer mit einem Spangen-Magnetelement versehenen Nagelspange auf einen zu korrigierenden Nagel einer Zehe oder eines Fingers. Nagelspangen sowie Nagelspangenapplikatoren sind bereits vorbekannt beispielsweise aus der EP 2 596 772 B1.

Dementsprechend ist es eine Aufgabe der vorliegenden Erfindung einen Nagelspangenapplikator hinsichtlich seiner Handhabbarkeit zu verbessern und ihn insbesondere benutzerfreundlicher auszugestalten.

Diese Aufgabe wird erfindungsgemäß gelöst durch einen Nagelspangenapplikator mit den im Anspruch 1 aufgeführten Merkmalen.

Die Grundkörper-Längsrichtung erstreckt sich dabei insbesondere in Richtung der längsten Ausdehnung des Grundkörpers des Nagelspangenapplikators. Die Grundkörper-Tiefenrichtung erstreckt sich insbesondere entlang der geringsten Ausdehnung des Nagelspangenapplikators. Die Grundkörper-Breitenrichtung steht insbesondere senkrecht sowohl auf der Grundkörper-Längsrichtung als auch auf der Grundkörper-Tiefenrichtung.

Durch die Wechselwirkung des Spangen-Magnetelements und des Applikator-Magnetelements wird die Nagelspange an der ersten Stirnseite des Nagelspangenapplikators gehalten, insbesondere befestigt.

Durch das magnetische Halten der Nagelspange an dem Nagelspangenapplikator, kann die Spange besonders effizient und benutzerfreundlich auf den Nagel aufgebracht werden. Es ist insbesondere nicht notwendig die Nagelspange selbstständig zu halten und/oder auszurichten. Hierdurch kann die Genauigkeit, mit der die Nagelspange auf den Nagel aufgebracht wird, verbessert werden. Insgesamt ist das Aufbringen der Nagelspange mit dem erfindungsgemäßen Nagelspangenapplikator nicht nur benutzerfreundlicher, sondern führt effektiv auch zu verbesserten medizinischen Ergebnissen.

Insbesondere erstreckt sich die konkave Anpress-Ausnehmung ausgehend von der ersten Grundkörper-Stirnseite in Grundkörper-Längsrichtung in den Grundkörper hinein und dehnt sich vorzugsweise in Grundkörper-Tiefenrichtung über die gesamte Tiefe des Grundkörpers aus.

Die konkave Auspressausnehmung kann dabei eine Mittelachse aufweisen. Die Mittelachse kann dabei insbesondere eine Symmetrieachse der konkaven Anpress-Ausnehmung darstellen. Es ist ebenfalls möglich, dass die konkave Anpress-Ausnehmung ohne inhärente Symmetrien ausgebildet ist.

Die konkave Anpress-Ausnehmung kann insbesondere als Ausschnitt einer Mantelfläche eines geraden Kreiszylinders geformt sein. In einem solchen Fall hat die konkave Anpress-Ausnehmung insbesondere einen konstanten Krümmungsradius und konstante Hauptkrümmungen.

Es ist ebenfalls möglich, dass die konkave Anpress-Ausnehmung als Teil der Mantelfläche eines allgemeinen Rotationskörpers, beispielsweise eines Rotationsellipsoids oder eines Rotationsparaboloids geformt ist. In einem solchen Fall wäre der Krümmungsradius der konkaven Anpress-Ausnehmung nicht konstant.

Durch die konkave Anpress-Ausnehmung wird eine Nagelspange bei ihrem Aufbringen auf den Nagel einer Zehe oder eines Fingers zumindest teilweise in die konkave Anpress-Ausnehmung hineingedrückt. Hierdurch verformt sich, die im Wesentlichen als quaderförmiger Körper ausgebildete, Nagelspange gemäß der Form der konkaven Anpress-Ausnehmung. Insbesondere nimmt die Nagelspange durch diese Verformung die Krümmung der konkaven Anpress-Ausnehmung an.

Idealerweise soll die Krümmung der Nagelspange in einem solchen Fall, zumindest teilweise, der Krümmung eines Zehen- bzw. Fingernagels entsprechen. Hierdurch lässt sich die Nagelspange besonders einfach und effizient auf dem zu behandelnden Nagel aufbringen.

Generell wird beim Aufbringen der Nagelspange auf einen Nagel der Nagel und/oder die Nagelspange mit einem Klebstoff behandelt, sodass die zwischen der Nagelspange und dem Nagel entstehende Klebekraft die magnetische Kraft zwischen dem Spangen-Magnetelement und dem Applikator-Magnetelement überkompensiert, wodurch sich die Nagelspange vom Nagelspangenapplikator löst.

Ohne eine erfindungsgemäße konkave Anpress-Ausnehmung gemäß Anspruch 1, muss ein Nagelspangenapplikator hierbei von Hand bogenförmig um die Zehe oder den Finger herumbewegt werden. Dies erfordert nicht nur einiges Geschick, sondern birgt auch das Risiko einer ungenauen und insbesondere einer fehlerhaften Aufbringung der Nagelspange auf dem Nagel, sodass die Nagelspange ihre gesundheitsfördernden und insbesondere heilenden Wirkungen nicht entfalten kann. Besagtes Geschick erforderndes Aufbringen entfällt mit einem Nagelspangenapplikator mit den Merkmalen des Anspruchs 1. Durch die Anpassung der Form der Nagelspange an die konkave Anpress-Ausnehmung passt sich die Form der Nagelspange auch an die Form des Nagels an. Die Nagelspange muss lediglich mit dem Applikator auf den Nagel aufgedrückt werden. Anschließend kann der Nagelspangenapplikator entfernt werden. Die Nagelspange wird hierdurch besonders benutzerfreundlich, einfach und vor allem sicher und effizient am Nagel befestigt und kann ihre volle Wirkung entfalten.

Ein Nagelspangenapplikator gemäß Anspruch 2 ist besonders einfach zu bedienen. Durch die Anordnung der konkaven Anpress-Ausnehmung in Breitenrichtung mittig an der ersten Grundkörper-Stirnseite des Grundkörpers muss ein Anwender des Nagelspangenapplikators keinen asymmetrischen Versatz von Nagelspange zum Nagelspangenapplikator eigenhändig ausgleichen.

Ein Nagelspangenapplikator gemäß Anspruch 3 ist besonders einfach herstellbar. Am Boden der konkaven Anpress-Ausnehmung ist eine sacklochartige Vertiefung, vorzugsweise mittig zu der konkaven Anpress-Ausnehmung in den Grundkörper des Nagelapplikators eingebracht. Unter einer mittigen Anordnung ist dabei insbesondere zu verstehen, dass der geometrische Flächenschwerpunkt der sacklochartigen Vertiefung und der geometrische Flächenschwerpunkt der konkaven Anpress-Ausnehmung zusammenfallen.

Die sacklochartige Vertiefung kann insbesondere als rechteckiges Sackloch ausgeführt sein. Es ist dabei möglich, dass die Ecken der rechteckigen Sacklochvertiefung abgerundet ausgestaltet sind. Auch andere Formen der sacklochartigen Vertiefung sind möglich.

Es ist ebenfalls möglich, dass die sacklochartige Vertiefung, zumindest abschnittsweise, eine rotationssymmetrische Form, insbesondere eine Zylinderform hat.

Die sachlochartige Vertiefung kann insbesondere mehrere Teilkammern aufweisen, die in Grundkörper-Breitenrichtung jeweils eine unterschiedliche Ausdehnung aufweisen können.

Es ist insbesondere möglich, dass die sacklochartige Vertiefung eine Magnetkammer und/oder eine Rückstellkammer aufweist. Es ist darüber hinaus möglich, dass die sacklochartige Vertiefung zu einer Öffnung der sacklochartigen Vertiefung hin eine Verjüngung aufweist.

Die Verjüngung kann insbesondere die kleinste Ausdehnung in Grundkörper-Breitenrichtung aufweisen. Die Magnetkammer kann insbesondere die größte Ausdehnung in Grundkörper-Breitenrichtung aufweisen. Die Ausdehnung der Rückstellkammer in Grundkörper-Breitenrichtung kann insbesondere zwischen der Ausdehnung der Verjüngung in Grundkörper-Breitenrichtung und der Ausdehnung der Magnetkammer in Grundkörper-Breitenrichtung liegen.

In die sacklochartige Vertiefung ist das Applikator-Magnetelement besonders leicht und effizient einbringbar. Das Applikator-Magnetelement kann dabei beispielsweise mittels Formschluss und/oder Stoffschluss in der sacklochartigen Vertiefung der konkaven Anpress-Ausnehmung gehalten werden.

Das Applikator-Magnetelement kann insbesondere in der Magnetkammer angeordnet sein. Das Applikator-Magnetelement kann insbesondere entlang der Grundkörper-Längsrichtung beweglich in der Magnetkammer angeordnet sein.

Die Nagelspange kann ihrerseits an ihrer der konkaven Anpress-Ausnehmung zugeordneten Seite ein ausgedehntes ferromagnetisches Element aufweisen, beispielsweise in Form eines flachen ferromagnetischen Plättchens. Es ist ebenfalls möglich, dass die Nagelspange an ihrer der konkaven Anpress-Ausnehmung zugerichteten Seite mit einem ferromagnetischen Lack beschichtet ist.

In beiden Fällen kann durch die Anordnung des Applikator-Magnetelements in der sacklochartigen Vertiefung die Magnetkraft zwischen dem Nagelspangenapplikator und der Nagelspange genau kontrolliert werden. Hierdurch kann präzise sichergestellt werden, dass die magnetische Wechselwirkung zwischen dem Nagelspangenapplikator und der Nagelspange so gewählt wird, dass die Nagelspange zum einen sicher vom Nagelspangenapplikator gehalten wird und sich zum anderen beim Aufbringen der Nagelspange auf einen Nagel problemlos lösen lässt.

Ein Nagelspangenapplikator gemäß Anspruch 4 ermöglicht es, die Magneten besonders effizient aufeinander abzustimmen. Es ist insbesondere nicht möglich, dass das Applikator-Magnetelement und das Spangen-Magnetelement einen physischen Kontakt zueinander herstellen, ohne dass eine äußere Kraft auf den Spangenapplikator und/oder gar die Spange einwirkt.

Vorzugsweise liegt ein im freien Zwischenraum vorhandener maximaler Abstand zwischen dem Spangen-Magnetelement und dem Applikator-Magnetelement im Bereich von 0,9 mm bis 3 mm, insbesondere 1 mm bis 2,8 mm, insbesondere 1,1 mm bis 2,6 mm, insbesondere 1,2 mm bis 2,4 mm, insbesondere 1,3 mm bis 2,2 mm, insbesondere 1,4 mm bis 2,0 mm und insbesondere 1,5 mm bis 1,8 mm. Dann ist eine gute magnetische Haltekraft gegeben.

Der freie Zwischenraum kann prinzipiell auch noch kleiner sein. Es ist insbesondere möglich, dass der freie Abstand kleiner ist als 1 mm, insbesondere kleiner als 0,5 mm, insbesondere kleiner als 0,3 mm und insbesondere kleiner als 0,1 mm. Es ist insbesondere auch möglich, dass das Spangen-Magnetelement und das Applikator-Magnetelement einen direkten physischen Kontakt ausbilden. Insgesamt steigt die magnetische Haltekraft mit einem kleiner werdenden freien Zwischenraum, so dass ein kleinerer freier Zwischenraum die Haltefähigkeit des Nagelspangenapplikators verbessert und damit dessen Benutzung erleichtert.

Ein Nagelspangenapplikator gemäß Anspruch 5 ist besonders benutzerfreundlich. Wenn jede der Auflageflächen sich bis zu einem der Stirnseiten-Querränder erstreckt, kann eine Nagelspange besonders einfach, entlang ihrer Längsausdehnung an der ersten Stirnseite des Grundkörpers des Nagelspangenapplikators befestigt werden. Außerdem kann die Nagelspange unterschiedliche Spangenlängen haben und insbesondere auch über die Stirnseiten-Querränder hinausstehen.

Die Stirnseiten-Längsränder können sich dabei insbesondere in Grundkörper-Breitenrichtung erstrecken und, zumindest im Bereich der konkaven Anpress-Ausnehmung gekrümmt verlaufen. Die Stirnseiten-Querränder können sich insbesondere in Grundkörper-Tiefenrichtung erstrecken.

Ein Nagelspangenapplikator gemäß Anspruch 6 ist besonders benutzerfreundlich. Die Führungselemente sind insbesondere als seitliche Führungselemente ausgebildet. Die Führungselemente sind insbesondere für alle Spangenlängen geeignet. Es ist dabei möglich, dass eine Spange in Grundkörper-Breitenrichtung über die erste Grundkörper-Stirnseite des Spangenapplikators übersteht.

Ein Nagelspangenapplikator gemäß Anspruch 7 erleichtert das Anbringen der Nagelspange an dem Nagelspangenapplikator merklich. Durch die Ausgestaltung der Führungselemente längs der Grundkörper-Breitenrichtung kann eine zu applizierende Nagelspange entlang ihrer Längsrichtung an der ersten Grundkörper-Stirnseite angebracht werden. Die Führungselemente garantieren dabei eine besonders einfache Führung der Nagelspange hin zu ihrer Applikationsposition an der ersten Grundkörper-Stirnseite. Die Führungselemente stellen dabei, entlang der Grundkörper-Tiefenrichtung, eine formschlüssige Verbindung mit der Nagelspange her. Es ist also nicht möglich, dass die Nagelspange in Grundkörper-Tiefenrichtung verrutscht oder verkippt, sodass die Nagelspange präzise an der ersten Grundkörper-Stirnseite des Nagelspangenapplikators angeordnet ist, was das Aufbringen der Nagelspange an einem Nagel erleichtert und verbessert.

Ein Nagelspangenapplikator gemäß Anspruch 8 ist besonders einfach herzustellen. Die Ausgestaltung der Führungselemente als Führungsstifte oder Führungsschienen ist problemlos mittels gängiger Materialbearbeitungsverfahren wie Drehen, Schleifen oder Fräsen herstellbar. Auch andere Herstellungsverfahren zur Herstellung der Führungsstifte oder Führungsschienen sind möglich.

Es ist auch möglich, dass der Nagelspangenapplikator und die Führungselemente als mehrteiliges Bauset ausgebildet sind, wobei jeweils alle Führungsstifte oder alle Führungsschienen als separate Bauteile ausgebildet sind. In einem solchen Fall wäre das Bauset fünfteilig. In einem solchen Fall sind Führungsstifte oder Führungsschienen ebenfalls besonders einfach mittels gängiger formgebender Verfahren beispielsweise dem Spritzgießen oder auch dem 3D-Druck herstellbar. Der Nagelspangenapplikator und die Führungselemente können beispielsweise mittels einer stoffschlüssigen Verbindung miteinander verbunden werden.

Ein Nagelspangenapplikator gemäß Anspruch 9 ist besonders vielseitig und flexibel einsetzbar. Durch die Kombination von sowohl Führungsschienen als auch von Führungsstiften die in Grundkörper-Tiefenrichtung gesehen zwei Aufnahmeabstände bilden, ist es möglich einen Nagelspangenapplikator als Kombinationsgerät für Nagelspangen verschiedener Breite zu verwenden. Gängige Nagelspangenbreiten sind dabei beispielsweise 3 mm oder auch 4 mm. Durch den Einsatz des Nagelspangenapplikators als Kombinationsgerät spart ein Bearbeiter darüber hinaus auch Arbeitszeit.

In einem solchen Fall können alle Führungsstifte und alle Führungsschienen als separates Bauteil ausgebildet sein, so dass das Bauset insgesamt neunteilig ist. Es ist auch möglich, dass jeweils ein Führungselement, umfassend sowohl eine Führungsschiene als auch einen Führungsstift als ein Bauteil ausgebildet ist. In einem solchen Fall umfasst das Bauset 5 Teile. Auch Mischformen, bei denen einzelne Führungselemente sowohl den Führungsstift als auch die Führungsschiene einteilig umfassen, während andere Führungsstifte und/oder Führungsschienen einteilig ausgebildet sind, ist möglich.

Ein Nagelspangenapplikator gemäß Anspruch 10 ermöglicht, mit den zwei voneinander beabstandeten Anpressspitzen schon mittig angebrachte Nagelspangen auch endgültig in den Randbereichen zu fixieren und/oder zu repositionieren. Der Nagelspangenapplikator lässt sich somit bevorzugt als Universalwerkzug bei praktisch allen Schritten zum Applizieren einer Nagelspange verwenden.

Ein Nagelspangenapplikator gemäß Anspruch 11 unterstützt den Anwender bei der Wahl einer geeigneten Nagelspange. Durch die an einer der Grundkörperlängsseiten angeordneten Messschablonen-Ausnehmungen kann die Größe eines Nagels vor dem Aufbringen einer Nagelspange vermessen und insbesondere bestimmt werden. Auf Grundlage dieser Vermessung kann eine Nagelspange passender Länge ausgewählt werden.

Dabei ist es besonders vorteilhaft, wenn der Nagelspangenapplikator entlang einer, besonders bevorzugt beider Grundkörperlängsseiten mehrere konkave Messschablonen-Ausnehmungen aufweist, die unterschiedliche Größe aufweisen. Der Nagelspangenapplikator kann dabei mindestens eine, insbesondere mindestens zwei, insbesondere mindestens drei, insbesondere mindestens vier, insbesondere mindestens fünf und insbesondere mindestens sechs Messschablonen-Ausnehmungen aufweisen. Auf diese Art kann ein Anwender beispielsweise mittels einer Heuristik wie "Trial-and-Error", die Nagelgröße sukzessive bestimmen und dadurch eine passende Nagelspange auswählen.

Besonders bevorzugt ist eine Ausgestaltung des Nagelspangenapplikators bei dem die konkaven Messschablonen-Ausnehmungen eine Beschriftung aufweisen. Diese Beschriftung kann beispielsweise eine Größenangabe in willkürlichen Einheiten darstellen, die dem Bearbeiter einen Hinweis auf die Größe des zu bearbeitenden Nagels gibt. Es ist auch möglich, dass die Markierung direkt mit der Länge, beispielsweise in Millimetern, der benötigten Nagelspange korreliert.

Ein Nagelspangenapplikator gemäß Anspruch 12 verbessert und erleichtert das Aufbringen von Nagelspangen auf einen Nagel merklich. Das Kennzeichnungs-Hilfsmittel kann dabei als sacklochartige Kennzeichnungs-Ausnehmung ausgestaltet sein. Durch die besagte sacklochartige Kennzeichnungs-Ausnehmung kann, beispielsweise mittels eines Stiftes, die Mitte des zu korrigierenden Nagels markiert werden.

Ein Nagelspangenapplikator gemäß Anspruch 13 erhöht die Präzision der Nagelspangenapplikation weiter. Durch die Mittenmarkierung des Applikationsbereichs des Nagelspangenapplikators ist es sehr einfach möglich die Nagelspange richtig zu positionieren, nämlich insbesondere dadurch, dass die Mittenmarkierung des Applikationsbereichs und die vorher am Nagel mittels der Messschablonen-Ausnehmung und deren Kennzeichnungs-Hilfsmittel auf dem Nagel angebrachte Mittenmarkierung übereinstimmen, insbesondere miteinander fluchten.

Ein Nagelspangenapplikator gemäß Anspruch 14 ist besonders benutzerfreundlich und bequem benutzbar. Durch die Anordnung des Applikator-Magnetelements an einer Rückstelleinrichtung ist es insbesondere möglich, dass das Applikator-Magnetelement in seiner insbesondere ausgefahrenen Ruhelage mit einer Oberfläche parallel und fluchtend zu mindestens einer der beiden Auflageflächen, und insbesondere zu beiden Auflageflächen angeordnet ist.

Das Applikator-Magnetelement ist insbesondere in Grundkörper-Längsrichtung beweglich ausgebildet. Es ist insbesondere möglich, dass das Applikator-Magnetelement innerhalb der sacklochartigen Vertiefung, in Grundkörper-Längsrichtung beweglich ausgebildet ist. Das Applikator-Magnetelement kann dabei insbesondere von seiner Ruhelage in eine vorgespannte Lage bewegt werden. Hierfür ist es insbesondere möglich, dass Führungselemente, wie beispielsweise Führungsschienen, an dem Applikator-Magnetelement angeordnet sind, die eine besonders reibungsarme Bewegung des Applikator-Magnetelements in Grundkörper-Längsrichtung ermöglichen.

Mit der Ruhelage des Applikator-Magnetelements sei dabei die Lage des Applikator-Magnetelements gemeint, die das Applikator-Magnetelement einnimmt, wenn keine äußere Kraft auf das Applikator-Magnetelement einwirkt. Dies ist insbesondere dann der Fall, wenn mittels des Nagelspangenapplikators nicht gerade eine Nagelspange auf einen zu behandelnden Finger- oder Zehennagel aufgebracht wird.

Mit der vorgespannten Lage sei dabei diejenige Lage des Applikator-Magnetelements gemeint, die das Applikator-Magnetelement bei insbesondere vollständiger Betätigung der Rückstelleinrichtung annimmt. Dies ist insbesondere dann der Fall, wenn eine Nagelspange mittels des Nagelspangenapplikators auf einen zu behandelnden Finger- oder Zehennagel angebracht wird.

Durch die parallele und fluchtende Anordnung der Oberfläche des Applikator-Magnetelements mit mindestens einer der beiden Auflageflächen ist es insbesondere möglich die Haltewirkung des Nagelspangenapplikators signifikant zu verbessern.

Die magnetische Anziehungskraft zwischen zwei Magnetelementen sinkt in der Regel rapide mit Vergrößerung des freien Zwischenraums zwischen den beiden Magnetelementen. Mittels der Rückstelleinrichtung ist es möglich, den freien Zwischenraum zwischen dem Applikator-Magnetelement und dem Spangen-Magnetelement zu reduzieren. Es ist insbesondere möglich, dass der freie Zwischenraum zwischen dem Applikator-Magnetelement und dem Spangen-Magnetelement kleiner ist als 1 mm, insbesondere kleiner als 0,5 mm, insbesondere kleiner als 0,3 mm und insbesondere kleiner als 0,1 mm. Es ist insbesondere auch möglich, dass das Applikator-Magnetelement und das Spangen-Magnetelement in direktem physischen Kontakt zueinander stehen, also insbesondere einander unmittelbar berühren. Dann gibt es keinen freien Zwischenraum.

Bei der mittels eines derartigen Nagelspangenapplikator erfolgenden Applikation einer Nagelspange auf einen Finger- oder Zehennagel wird eine Kraft auf die Rückstelleinrichtung ausgeübt, die einer Rückstellkraft der Rückstelleinrichtung entgegenwirkt. Hierdurch wird die Rückstelleinrichtung betätigt, wodurch das Applikator-Magnetelement, zumindest teilweise, in die konkave Anpress-Ausnehmung, und insbesondere in die sacklochartige Vertiefung, hineingedrückt wird. Die Nagelspange kann sich bei der Applikation also weiterhin der charakteristischen Form des Nagels anpassen.

Es ist insbesondere möglich, dass die Rückstelleinrichtung, zumindest teilweise und insbesondere vollständig in der sacklochartigen Vertiefung angeordnet ist. Hierfür kann die sacklochartige Vertiefung eine Rückstellkammer aufweisen.

Es ist möglich, dass die sacklochartige Vertiefung eine Magnetkammer aufweist, in der das Applikator-Magnetelement, zumindest teilweise, und insbesondere vollständig angeordnet ist. Die Magnetkammer kann insbesondere unmittelbar an die Rückstellkammer angrenzen.

Es ist insbesondere möglich, dass die Rückstellkammer eine Rückstell-Ausdehnung in Grundkörper-Breitenrichtung aufweist, die geringer ist als eine Magnet-Ausdehnung der Magnetkammer in Grundkörper-Breitenrichtung. Hierdurch kann beim Übergang von der Magnetkammer zur Rückstellkammer insbesondere eine Kante entstehen.

Es ist insbesondere möglich, dass das Applikator-Magnetelement einen Magnet-Längskörper und einen Magnet-Breitenkörper aufweist. Der Magnet-Längskörper und der Magnet-Breitenkörper können insbesondere einstückig ausgebildet sein. Das Applikator-Magnetelement kann insbesondere eine T-förmige Längsschnittfläche aufweisen. Senkrecht dazu kann das Applikator-Magnetelement insbesondere eine eckige, vorzugsweise rechteckige, oder eine runde Querschnittsfläche haben. Das Applikator-Magnetelement kann zumindest abschnittsweise, insbesondere eine Quaderform oder eine rotationssymmetrische Form, vorzugsweise eine Zylinderform, haben.

Der Magnet-Breitenkörper weist insbesondere in Grundkörper-Breitenrichtung eine größere Ausdehnung auf als der Magnet-Längskörper. Der Magnet-Breitenkörper ist insbesondere vollständig innerhalb der Magnetkammer angeordnet. Beim Betätigen der Rückstelleinrichtung kann der Magnet-Breitenkörper einen vorgegebenen Hub entlang der Grundkörper-Längsrichtung durchlaufen, bis der Magnet-Breitenkörper an der Kante die den Übergang von der Magnetkammer zur Rückstellkammer markiert anschlägt. Dieser Anschlag stellt einen unteren Bewegungsendpunkt des Magnet-Breitenkörpers dar. Beim Anschlag des Magnet-Breitenkrörpers an der Kante erreicht das Applikator-Magnetelement insbesondere seine vorgespannte Lage. Es ist insbesondere möglich, dass die Oberfläche des Applikator-Magnetelements. die in Ruhelage parallel und fluchtend zu mindestens einer der beiden Anlageflächen angeordnet ist, am unteren Bewegungsendpunkt des Applikator-Magnetelements parallel und fluchtend zu der Öffnung der sacklochartigen Vertiefung angeordnet ist.

Die sacklochartige Vertiefung kann insbesondere zu ihrer Öffnung hin verjüngend ausgebildet sein. Es ist insbesondere möglich, dass zwischen der Magnetkammer und einer Verjüngung der sacklochartigen Vertiefung eine weitere Kante ausgebildet ist, an welcher der Magnet-Breitenkörper in der Ruhelage anliegt.

Ein Nagelspangenapplikator nach Anspruch 15 ist besonders einfach zu konstruieren. Ein Rückstellelement stellt die einfachste Möglichkeit dar, eine Rückstelleinrichtung, mit einer zugehörigen Rückstellkraft, zu realisieren.

Es ist insbesondere möglich, dass die Rückstelleinrichtung mindestens zwei Rückstellelemente und insbesondere mindestens drei Rückstellelemente aufweist. Durch eine höhere Anzahl an Rückstellelementen verteilt sich die Kraft, die auf die Rückstelleinrichtung bei Applikation einer Nagelspange einwirkt, insbesondere gleichmäßig auf alle Rückstellelemente. Hierdurch kann die Abnutzung einzelner Rückstellelemente bzw. ein Materialversagen einzelner Rückstellelemente vermieden werden.

Die Rückstellelemente können beispielsweise als Federn ausgebildet sein. Es ist insbesondere möglich, dass die Rückstellelemente als Blattfedern ausgebildet sind.

Die Rückstellelemente können insbesondere auch als Federringe, vorzugsweise aus einem Kunststoff, ausgebildet sein. Weiterhin können die Rückstellelemente insbesondere einstückig miteinander verbunden sein.

Zudem kann die Rückstelleinrichtung vorzugsweise ein einteiliges Kunststoff-Bauteil sein, das ein federndes Rückstellelement, oder zwei oder mehr federnde Rückstellelemente, aufweist, das insbesondere einstückig gegossen ist.

Bei der Verwendung von mehr als einem Rückstellelement können alle Rückstellelemente identisch zueinander ausgebildet sein. Es ist auch möglich, dass verschiedene Rückstellelemente verschiedene Eigenschaften besitzen. Es ist beispielsweise möglich drei Federringe mit unterschiedlichen elastischen Eigenschaften zu verwenden. Auch die Kombination von Federringen und Blattfedern ist möglich.

Nachfolgend wird beispielhaft ein Verfahren zur Applikation einer mit einem Spangen-Magnetelement versehenen Nagelspange auf einen zu korrigierenden Nagel einer Zehe oder eines Fingers mit einem erfindungsgemäßen Nagelspangenapplikator beschrieben. Das Verfahren umfasst die folgenden Schritte: Auswahl einer zu applizierenden Nagelspange mit geeigneter Spangen-Länge und geeigneter Spangen-Breite, Fixieren der zu applizierenden Nagelspange an der ersten Stirnseite des Nagelspangenapplikators, wobei die Nagelspange mittels magnetischer Wechselwirkung gehalten wird, Aufbringen eines Klebstoffs auf dem zu korrigierenden Nagel und/oder auf der, der konkaven Anpress-Ausnehmung abgewandten, Seite der Nagelspange, Andrücken der Nagelspange mit dem Nagelspangenapplikator auf dem zu korrigierenden Nagel, wobei die Nagelspange, zumindest teilweise, in die konkave Anpress-Ausnehmung des Nagelspangenapplikators hineingedrückt wird und sich dadurch, zumindest teilweise, der Form des zu korrigierenden Nagels anpasst, Entfernen des Nagelspangenapplikators von dem zu korrigierenden Nagel, wobei durch die adhäsive und/oder kohäsive Wechselwirkung der Nagelspange und/oder des Nagels mit dem Klebstoff die Nagelspange von dem Nagelspangenapplikator gelöst und am Nagel fixiert wird.

Weiterhin kann das Verfahren einen Schritt umfassen, nach dem, mit einem Nagelspangenapplikator gemäß einem der Ansprüche 11 oder 12, die Auswahl einer Nagelspange mit geeigneter Spangen-Länge und geeigneter Spangen-Breite, das Vermessen der Zehe mit der mindestens einen konkaven Messschablonen-Ausnehmung umfasst.

Weiterhin kann das Verfahren einen Schritt umfassen, nach dem mit einem Nagelspangenapplikator gemäß Anspruch 12 mit dem Kennzeichnungs-Hilfsmittel die Mitte der Zehe markiert wird, beispielsweise mit einem abwaschwaren Stift wie einem Bleistift.

Weiterhin kann das Verfahren einen Schritt umfassen, nach dem mit einem Nagelspangenapplikator gemäß Anspruch 13 die Mittenmarkierung des Nagelspangenapplikators fluchtend zu der Mittenmarkierung des Nagels angeordnet wird.

Weiterhin kann das Verfahren einen Schritt umfassen, nach dem mit einem Nagelspangenapplikator gemäß dem Anspruch 9 eine bereits auf dem Nagel fixierte Nagelspange an ihren Randbereichen mittels der Anpressspitzen der zweiten Stirnseite des Nagelspangenapplikators zusätzlich angedrückt und/oder repositioniert wird. Hierdurch kann sichergestellt werden, dass die Nagelspange vollständig und gleichmäßig an dem Nagel angebracht ist.

Im Folgenden werden unter Bezugnahme auf die Figuren weitere Einzelheiten, Vorteile und Merkmale der Erfindung beschrieben. Dabei zeigen:
- Fig. 1: ein Ausführungsbeispiel für einen Nagelspangenapplikator in einer perspektivischen Ansicht;
- Fig. 2: ein Ausführungsbeispiel für den Nagelspangenapplikator nach Fig. 1 in einer Seitenansicht;
- Fig. 3: ein Ausführungsbeispiel für den Nagelspangenapplikator nach Fig. 1 in einer Frontansicht;
- Fig. 4: eine perspektivische-Ansicht eines Ausschnitts des Nagelspangenapplikators nach Fig. 1, bei dem die erste Grundkörper-Stirnseite vergrößert dargestellt ist;
- Fig. 5: eine vergrößerte Seitenansicht eines Ausschnitts des Nagelspangenapplikators nach Fig. 1 mit einer Nagelspange;
- Fig. 6: eine Draufsicht der Applikation einer Nagelspange auf einem Nagel mithilfe eines Nagelspangenapplikators gemäß Fig. 1; und
- Fig. 7: eine Schnittdarstellung des Applikationsbereichs eines weiteren Ausführungsbeispiels eines Nagelspangenapplikators mit einem längs beweglichen Applikator-Magnetelement und einer Rückstelleinrichtung.

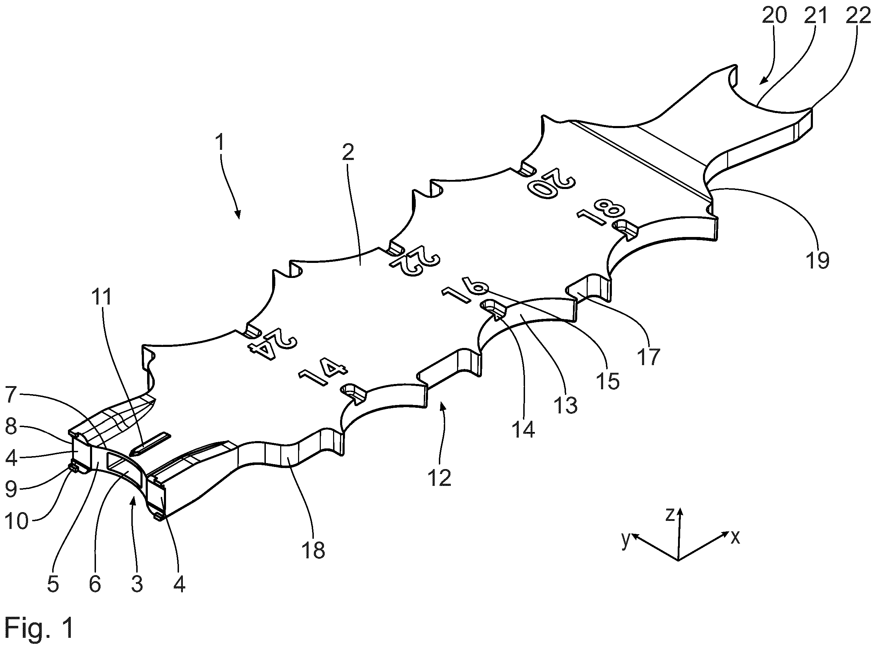

Einander entsprechende Teile sind in den Fig. 1 bis 6 mit denselben Bezugszeichen versehen. Auch Einzelheiten des im Folgenden näher erläuterten Ausführungsbeispiels können für sich genommen eine Erfindung darstellen oder Teil eines Erfindungsgegenstandes sein.

Fig. 1 zeigt einen Nagelspangenapplikator 1. Der Nagelspangenapplikator 1 weist einen, sich entlang seiner Längsachse in einer Grundkörper-Längsrichtung x erstreckenden Grundkörper 2 auf. Der Grundkörper 2 weist eine erste Grundkörper-Stirnseite 3, die einen Applikationsbereich ausbildet, auf.

Die erste Grundkörper-Stirnseite 3 umfasst zwei rechteckige Auflageflächen 4 und eine konkave Anpress-Ausnehmung 5.

Die konkave Anpress-Ausnehmung 5 erstreckt sich in den Grundkörper 2 des Nagelspangenapplikators 1 hinein.

Die Auflageflächen 4 sind eben und haben insbesondere keinerlei Krümmung. Die Auflageflächen 4 sind im Wesentlichen senkrecht zur Grundkörper-Längsrichtung x des Grundkörper 2 des Nagelspangenapplikators 1 ausgedehnt. Die konkave Anpress-Ausnehmung 5 ist zwischen den zwei Auflageflächen 4 angeordnet.

Die konkave Anpress-Ausnehmung 5 weist an ihrem Boden eine sacklochartige Vertiefung 6 auf. Die sacklochartige Vertiefung 6 dient dazu ein, nicht in der Fig. 1 dargestelltes, Applikator-Magnetelement, zumindest teilweise, aufzunehmen.

Die erste Grundkörper-Stirnseite 3 weist zwei einander in einer Grundkröper-Tiefenrichtung z gegenüberliegende Längsränder 7 und zwei einander in einer Grundkörper-Breitenrichtung y gegenüberliegende Querränder 8 auf. Der Längsrand 7 verläuft dabei im Wesentlichen entlang der größeren Ausdehnung der ersten Grundkörper-Stirnseite 3 in Grundkörper-Breitenrichtung y. Der Querrand 8 verläuft entlang der kleineren Ausdehnung der Grundkörper-Stirnseite 3 in Grundkörper-Tiefenrichtung z. Der Längsrand 7 verläuft dabei im Bereich der konkaven Anpress-Ausnehmung 5 gekrümmt.

Entlang des Längsrandes 7 erstrecken sich Führungselemente 9, 10, die das Anordnen einer, nicht in der Fig. 1 dargestellten, Nagelspange am Applikationsbereich des Nagelspangenapplikators 1 erleichtert. Die Führungselemente 9, 10 erleichtern insbesondere das Anbringen der Nagelspange entlang deren Längsrichtung am Nagelspangenapplikator 1. Die Führungselemente 9, 10 sind insbesondere dazu ausgebildet eine am Nagelspangenapplikator 1 angebrachte Nagelspange entlang der Grundkörper-Tiefenrichtung z formschlüssig zu halten. Die Nagelspange ist hierdurch gegen ein Verrutschen oder ein Verkippen entlang der Grundkörper-Tiefenrichtung z geschützt.

Die Führungselemente 9, 10 sind im in der Fig. 1 dargestellten Ausführungsbeispiel teils als Führungsschienen 9 und teils als Führungsstifte 10 ausgebildet. Es ist auch möglich, dass ein Nagelspangenapplikator 1 an seiner ersten Grundkörper-Stirnseite 3 entweder nur Führungsschienen 9 oder nur Führungsstifte 10 aufweist.

Der Nagelspangenapplikator 1 weist insgesamt vier Führungsschienen 9 und vier Führungsstifte 10 auf. Die Führungsschienen 9 und die Führungsstifte 10 sind jeweils paarweise an den Querrändern 8 der ersten Grundkörper-Stirnseite angeordnet und erstrecken sich in Richtung des Längsrandes 7. Pro Anlagefläche 4 sind dabei jeweils zwei Führungsschienen 9 und zwei Führungsstifte 10 angeordnet.

Die Führungsschienen 9 weisen sowohl in Grundkörper-Tiefenrichtung z als auch in Grundkörper-Breitenrichtung y eine größere Ausdehnung auf als die Führungsstifte 10. Die Führungsstifte 10 weisen in Grundkörper-Längsrichtung x eine größere Ausdehnung auf als die Führungsschiene 9. Hierdurch ist es insbesondere möglich den Nagelspangenapplikator 1 für das Aufbringen von Nagelspangen unterschiedlicher Breite zu verwenden. Breitere Nagelspangen können durch die Führungsstifte 10 gelagert werden. Schmalere Nagelspangen können durch die Führungsschiene 9 gelagert werden. Gängige Breiten für Nagelspangen sind dabei 3 mm und 4 mm. Die Führungsschienen 9 und die Führungsstifte 10 sind auf diese jeweiligen Abstände abgestimmt.

Im Bereich der ersten Grundkörper-Stirnseite 3 weist der Grundkörper 2 des Nagelspangenapplikators 1 eine Mittelmarkierung 11 auf. Die Mittelmarkierung 11 ist als pfeilförmige Markierung ausgebildet. Die pfeilförmige Markierung umfasst dabei einen rechteckigen Markierungs-Grundkörper und eine dreiecksförmige Spitze. Die Spitze zeigt zum Längsrand 7 der ersten Grundkörper-Stirnseite 3.

Die Mittelmarkierung 11 ist zentral zwischen den zwei Querrändern 8 angeordnet. Insbesondere die Spitze der Mittelmarkierung 11 zeigt exakt auf die Mitte der konkaven Anpress-Ausnehmung 5 der ersten Grundkörper-Stirnseite 3. Dadurch, dass die konkave Anpress-Ausnehmung 5 zentral in der ersten Grundkörper-Stirnseite 3 angeordnet ist, zeigt die Spitze der Mittelmarkierung 11 somit auch die exakte Mitte der ersten Grundkörper-Stirnseite 3 an.

Weiterhin zeigt die Fig. 1, dass der Grundkörper 2 des Nagelspangenapplikators 1 zwei Grundkörper-Längsseiten 12 aufweist. Die Grundkörpe-Längsseiten 12 sind dabei die längsten Seiten des Nagelspangenapplikators 1 und erstrecken sich entlang der Grundkörper-Längsrichtung x.

Die Grundkörperlängsseiten 12 weisen mehrere konkave Messschablonen-Ausnehmungen 13 auf. Bei dem in Fig. 1 gezeigten Ausführungsbeispiel weisen beide Grundkörper-Längsseiten 12 jeweils drei konkave Messschablonen-Ausnehmungen 13 auf. Die konkaven Messschablonen-Ausnehmungen 13 erstrecken sich in Grundkörper-Breitenrichtung y in das Innere des Grundkörpers 2 des Nagelspangenapplikators 1 hinein.

Jede der konkaven Messschablonen-Ausnehmungen 13 weist an ihrem jeweiligen Boden ein mittig angeordnetes Kennzeichnungs-Hilfsmittel 14 auf. Das Kennzeichnungs-Hilfsmittel 14 ist bei dem in Fig. 1 gezeigten Ausführungsbeispiel als sacklochartige Vertiefung ausgeführt. Die Kennzeichnungs-Hilfsmittel 14 erstrecken sich in Grundkörper-Breitenrichtung y in das Innere des Grundkörpers 2 hinein. Dabei sind die Kennzeichnungs-Hilfsmittel 14 weder in Grundkörper-Breitenrichtung y noch in Grundkörper-Tiefenrichtung z als Durchgangsöffnungen ausgebildet. Die Kennzeichnungs-Hilfsmittel 14 weisen, sowohl in Draufsicht als auch in Seitenansicht, eine im Wesentlichen rechteckige Kontur auf.

Zu jeder der konkaven Messschablonen-Ausnehmungen 13 ist am Grundkörper 2 des Nagelspangenapplikators 1 eine Markierung 15 angebracht. In dem von Fig. 1 gezeigten Ausführungsbeispiel sind entsprechend sechs solcher Markierungen 15 angebracht. Die Markierungen 15 sind als arabische Ziffern "14", "16", "18", "20", "22", und "24" ausgebildet. Die Markierungen 15 geben jeweils eine Länge der zu applizierenden Nagelspange an. Generell gilt dabei, dass eine Messschablonen-Ausnehmung 13, deren Markierung 15 von einer größeren arabischen Ziffer dargestellt wird, mit einer längeren Nagelspange korreliert. Die Markierungen 15 können dabei in willkürlichen Einheiten einen Hinweis auf die Länge der zu applizierenden Nagelspange geben oder direkt der Länge der Nagelspange, beispielsweise in Millimetern, entsprechen.

Jede der konkaven Messschablonen-Ausnehmungen 13 weist zwei Ränder auf. Die Ränder schließen die jeweilige Messschablonen-Ausnehmung 13 in Grundkörper-Längsrichtung x ab. An eine konkave Messschablonen-Ausnehmung 13 grenzt nicht direkt eine weitere konkave Messschablonen-Ausnehmung 13. Vielmehr ist zwischen zwei Messschablonen-Ausnehmungen 13 ein Zwischenraum 17 angeordnet.

Die Zwischenräume 17 sind im Ausführungsbeispiel gemäß Fig. 1 als Einbuchtungen entlang der Grundkörper-Breitenrichtung y in den Grundkörper 2 des Nagelspangenapplikators 1 hinein ausgebildet.

Der Grundkörper 2 weist weiterhin zwei Kurven 18, 19 auf. Mit der ersten Kurve 18 wird derjenige Teil des Grundkörpers 2, der die konkaven Messschablonen-Ausnehmungen 13 enthält, von der bereits beschriebenen ersten Stirnfläche 3 und dem damit einhergehenden Applikationsbereich getrennt. Im Bereich der ersten Kurve 18 wird die Ausdehnung des Grundkörpers 2 entlang der Grundkörper-Breitenrichtung y geringer.

Die zweite Kurve 19 verbindet denjenigen Teil des Grundkörpers 2, der die konkaven Messschablonen-Ausnehmungen 13 aufweist, mit einer zweiten Stirnseite 20. Auch die zweite Kurve 19 führt zu einer Reduzierung der Ausdehnung des Grundkörpers 2 entlang der Grundkörper-Breitenrichtung y.

Die zweite Stirnseite 20 weist eine konkave Ausnehmung 21 auf. Die Ränder der konkaven Ausnehmung 21 sind jeweils als Anpressspitzen 22 ausgebildet. Mittels dieser Anpressspitzen 22 ist es möglich, eine bereits zum Teil auf einem Nagel aufgebrachte Nagelspange zusätzlich anzudrücken und somit zu stabilisieren.

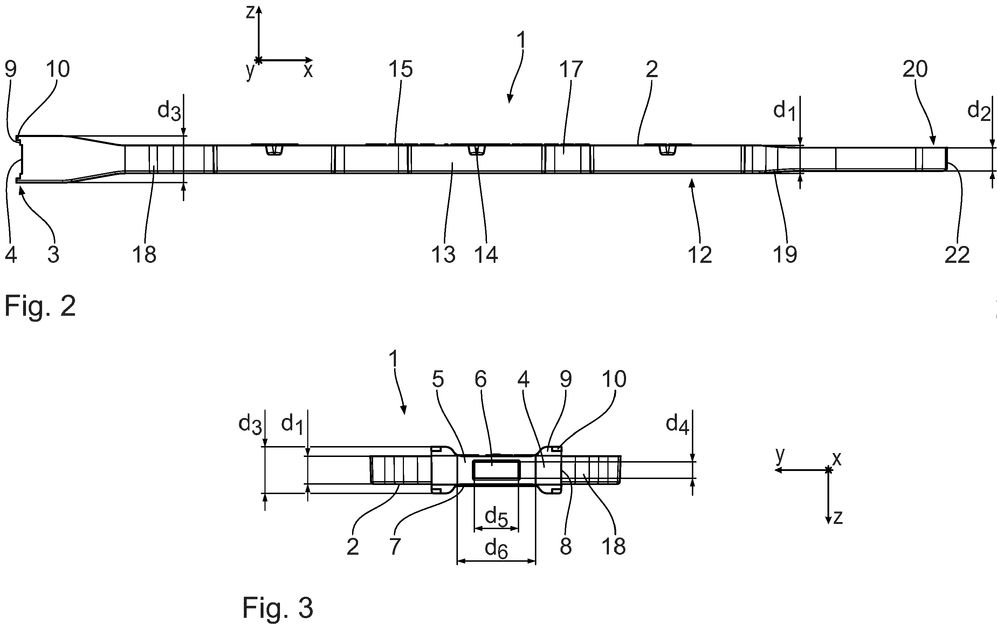

Fig. 2 zeigt einen Nagelspangenapplikator 1 gemäß dem Ausführungsbeispiel von Fig. 1 in Seitenansicht. Die Blickrichtung ist dabei die Grundkörper-Breitenrichtung y. Entsprechend ist in Fig. 2 die Grundkörperlängsseite 12 dargestellt. An den beiden Enden der Grundkörperlängsseite 12 befinden sich die erste Grundkörper-Stirnseite 3 und die zweite Grundkörper-Stirnseite 20.

Der Nagelspangenapplikator weist in Grundkörper-Tiefenrichtung z eine Haupttiefe d₁ auf. Zur ersten Grundkörper-Stirnseite 3 hin erhöht sich die Ausdehnung des Grundkörpers 2 des Nagelspangenapplikators 1 entlang der Grundkörper-Tiefenrichtung z kontinuierlich bis zu einer Tiefe d₃. Die Tiefe d₃ beschreibt insbesondere die größte Ausdehnung des Nagelspangenapplikators 1 in Grundkörper-Tiefenrichtung z.

Entlang der zweiten Kurve 19 verkleinert sich die Ausdehnung des Grundkörpers 2 entlang der Grundkörper-Tiefenrichtung z kontinuierlich zu einer Tiefe d₂. Die Tiefe d₂ beschreibt insbesondere die kleinste Ausdehnung des Nagelspangenapplikators 1 in Grundkörper-Tiefenrichtung z.

Für die Haupttiefe d₁ kann insbesondere gelten: 3 mm ≤ d₁ ≤ 5 mm. Insbesondere kann die Haupttiefe d₁ auch exakt 4 mm betragen. Für die Tiefe d₂ kann insbesondere gelten: 2 mm ≤ d₂ ≤ 4 mm. Insbesondere kann die Tiefe d₂ auch exakt 3 mm betragen. Für die Tiefe d₃ kann insbesondere gelten: 4 mm ≤ d₃ ≤ 6 mm. Insbesondere kann die Tiefe d₃ auch exakt 5 mm betragen.

Fig. 3 zeigt eine Frontansicht der ersten Grundkörper-Stirnseite 3 mit Blickrichtung in Grundkörper-Längsrichtung x. Neben einer nochmaligen Darstellung der Tiefen d₁ und d₃ ist in dieser Figur die Vertiefung 6, die das Applikator-Magnetelement zumindest teilweise enthält näher dargestellt. Entlang der Grundkörper-Tiefenrichtung z weist die Vertiefung 6 eine Tiefen-Ausdehnung d₄ auf. Entlang der Grundkörper-Breitenrichtung y weist die Vertiefung 6 eine Breiten-Ausdehnung d₅ auf. Die Tiefen-Ausdehnung d₄ ist insbesondere kleiner als die Haupttiefe d₁.

Weiterhin zeigt die Fig. 3, dass die konkave Anpress-Ausnehmung 5 eine Breiten-Ausdehnung d₆ in Grundkörper-Breitenrichtung aufweist, die insbesondere größer ist als die Breiten-Ausdehnung d₅ der Vertiefung 6.

Für die Tiefen-Ausdehnung d₄ kann insbesondere gelten: 1 mm ≤ d₄ ≤ 3 mm. Insbesondere kann die Tiefen-Ausdehnung d₄ auch exakt 2 mm betragen. Für die Breiten-Ausdehnung d₅ kann insbesondere gelten: 4 mm ≤ d₅ ≤ 6 mm. Insbesondere kann die Breiten-Ausdehnung d₅ auch exakt 5 mm betragen. Für die Breiten-Ausdehnung d₆ kann insbesondere gelten: 8 mm ≤ d₆ ≤ 10 mm. Insbesondere kann die Breiten-Ausdehnung d₆ exakt 9 mm betragen.

Die Vertiefung 6 ist insbesondere zentriert innerhalb der konkaven Anpress-Ausnehmung 5 angeordnet. Gemäß der in Fig. 3 dargestellten Frontansicht fallen die geometrischen Schwerpunkte der Vertiefung 6 und der konkaven Anpress-Ausnehmung 5 zusammen.

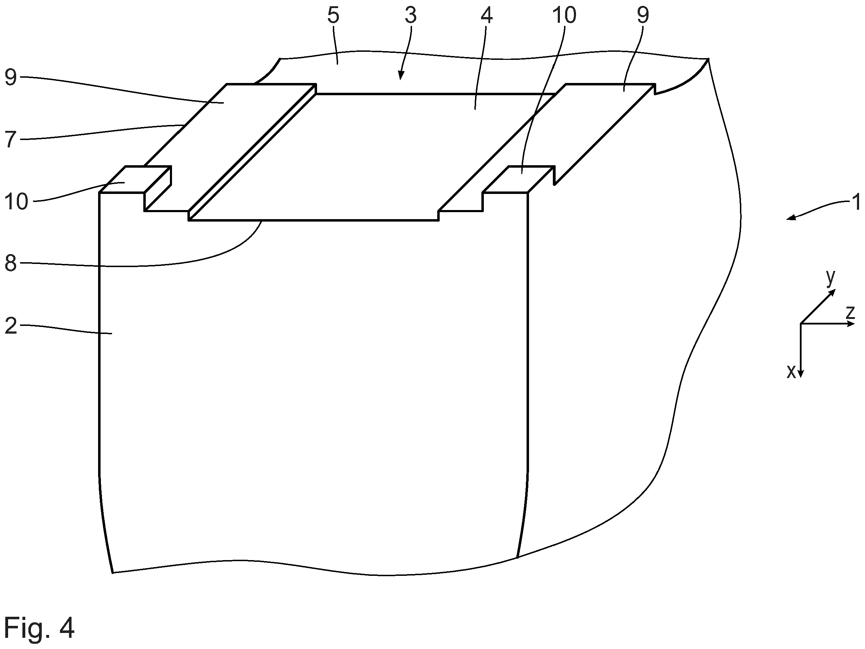

Fig. 4 zeigt einen dreidimensionalen Ausschnitt des Grundkörpers 2 im Bereich der ersten Grundkörper-Stirnseite 3. In Grundkörper-Breitenrichtung y ist der Ausschnitt unvollständig. Insbesondere sind nur zwei der Führungsstifte 10 sowie zwei der Führungsschienen 9 von einer der Auflageflächen 4 dargestellt.

Entlang der Grundkörper-Breitenrichtung y ist eine Nagelspange entlang deren Längsrichtung an dem Nagelspangenapplikator 1 befestigbar. Die Breite der Nagelspange kann dabei insbesondere so gewählt werden, dass die Bewegung der Nagelspange entlang der Grundkörper-Tiefenrichtung z von wahlweise den Führungsschienen 10 oder den Führungsstiften 9 formschlüssig begrenzt wird.

Hierfür stehen die Führungsstifte 10 entgegen der Grundkörper-Längsrichtung x über die Führungsschienen 9 hinaus. Die Führungsschienen 9 weisen in Grundkörper-Tiefenrichtung z und/oder in Grundkörper-Breitenrichtung y eine größere Ausdehnung auf als die Führungsstifte 10. Eine Nagelspange wird so platziert, dass ihre Breite längs der Grundkörper-Tiefenrichtung z orientiert ist. Auf diese Weise eignen sich die Anlageflächen 4 zum Lagern von Nagelspangen unterschiedlicher Breite.

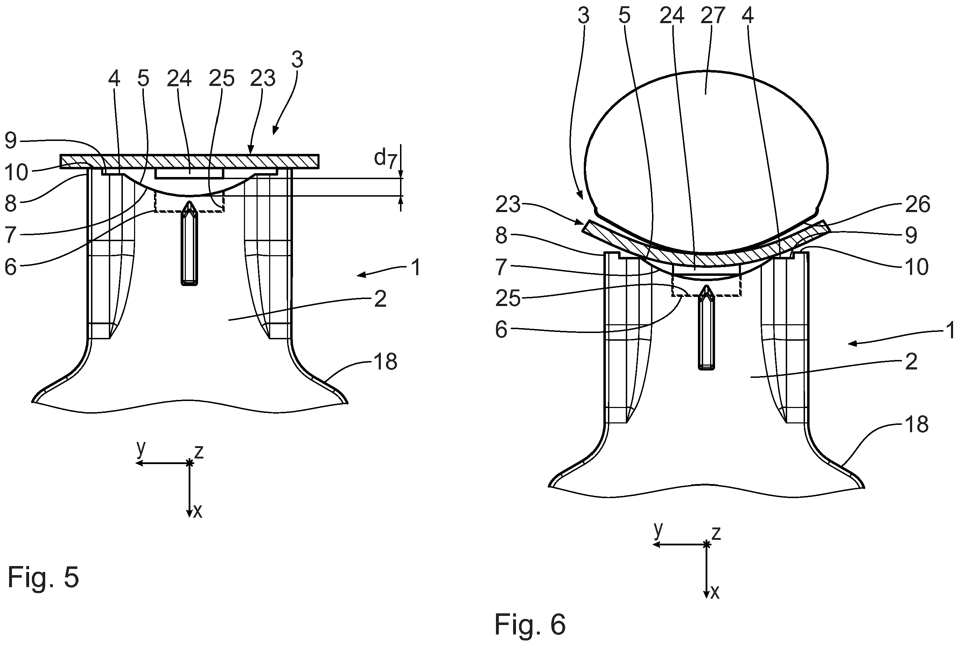

Fig. 5 zeigt eine ausschnittsweise Seitenansicht eines Nagelspangenapplikators 1 gemäß dem Ausführungsbeispiel nach Fig. 1 mit einer Nagelspange 23 kurz vor dem Einsetzen in den Nagelspangenapplikator 1. Durch die gewählte Darstellung der Nagelspange 23 sind die Führungsstifte 10 bzw. die Führungsschienen 9 nach wie vor erkennbar. Weiterhin weist die Nagelspange 23 ein Spangen-Magnetelement 24 auf.

Der Nagelspangenapplikator 1 weist ein Applikator-Magnetelement 25 auf, welches zumindest teilweise in der, nicht in der Fig. 5 dargestellten, Vertiefung 6 angeordnet ist.

In Grundkörper-Längsrichtung x ist zwischen dem Spangen-Magnetelement 24 und dem Applikator-Magnetelement 25 ein freier Abstand d₇ vorhanden. Das Spangen-Magnetelement 24 und das Applikator-Magnetelement 25 stehen über den freien Abstand d₇ hinweg in magnetischer Wechselwirkung zueinander, so dass die Nagelspange 23 an der ersten Grundkörper-Stirnseite, insbesondere lösbar, fixiert ist.

Vorzugsweise liegt der freie Abstand d₇ zwischen dem Spangen-Magnetelement und dem Applikator-Magnetelement im Bereich von 0,9 mm bis 3 mm, insbesondere 1 mm bis 2,8 mm, insbesondere 1,1 mm bis 2,6 mm, insbesondere 1,2 mm bis 2,4 mm, insbesondere 1,3 mm bis 2,2 mm, insbesondere 1,4 mm bis 2,0 mm und insbesondere 1,5 mm bis 1,8 mm. Der freie Abstand d₇ kann auch exakt 1,6 mm betragen

Die Nagelspange 23 kann in Grundkörper-Breitenrichtung y eine größere Ausdehnung aufweisen als die erste Grundkörper-Stirnseite 3. Hierdurch steht die Nagelspange 23 entlang ihrer Längsrichtung, in Grundkörper-Breitenrichtung y, auf beiden Seiten über die erste Grundkörper-Stirnseite 3 über.

Entlang der Grundkörper-Tiefenrichtung z ist die Nagelspange 23 formschlüssig gehalten, bei dem in Fig. 5 dargestellten Ausführungsbeispiel, von den Führungsstiften 10. Es ist ebenso möglich, dass eine Nagelspange 23 von den Führungsschienen 9 gehalten wird.

Fig. 6 zeigt in Draufsicht einen Teil des Nagelspangenapplikators 1 mit einer Nagelspange 23, welche gerade auf einen Nagel 26 eines Körperteils 27, welches beispielsweise ein Finger oder eine Zehe sein kann, aufgebracht wird. Beim Aufbringen der Nagelspange 23 auf einen Nagel 26 wird die Nagelspange 23 durch den bei dem Aufbringen ausgeübten Druck in die konkave Anpress-Ausnehmung 5 hinein gedrückt und verformt sich dabei ebenfalls konkav. Die Nagelspange 23 nimmt dabei im Wesentlichen dieselbe Form an wie die konkave Anpress-Ausnehmung 5. Es ist möglich die Nagelspange 23 so weit in die konkave Anpress-Ausnehmung 5 hineinzudrücken, bis sich die beiden Magnetelemente 24, 25 berühren. Hierdurch passt sich die Nagelspange 23 auf natürliche Weise der Krümmung des Nagels 26 an, was das Aufbringen der Nagelspange 23 auf dem Nagel 26 erleichtert.

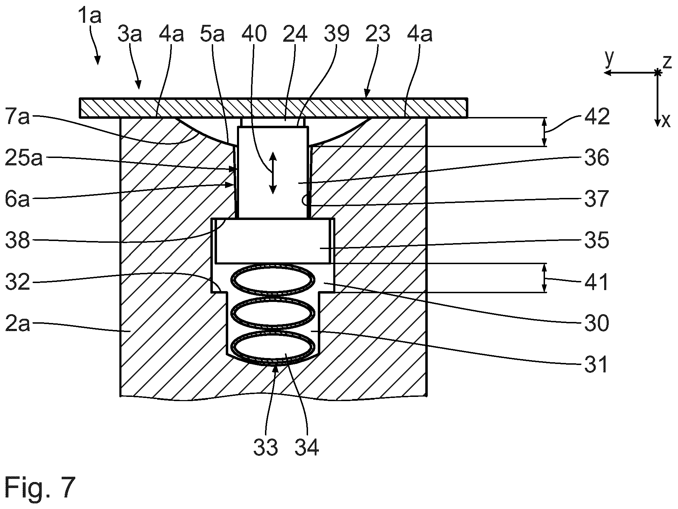

Fig. 7 zeigt in Schnittdarstellung ein weiteres Ausführungsbeispiel eines Nagelspangenapplikators 1a. Die Figur zeigt den Applikationsbereich des Nagelspangenapplikators 1a mit eingesetzter Nagelspange 23, sodass die Führungsstifte 10 und die Führungsschienen 9 in der Fig. 7 nicht erkennbar sind.

Zu dem Nagelspangenapplikator 1 gemäß dem ersten Ausführungsbeispiel äquivalente Bauteile werden mit gleichen Bezugsziffern und dem Zusatz "a" referenziert und im Folgenden nicht erneut beschrieben.

In der Fig. 7 ist insbesondere eine schmale Nagelspange 23 (Breite 3 mm) in den Nagelspangenapplikator 1a eingesetzt, die dabei auf den Auflageflächen 10 der ersten Grundkörper-Stirnseite 3a des länglichen Grundkörpers 2a aufliegt, und deren Orientierung durch die Führungsschienen 9 vorgegeben wird.

Wie bereits erläutert, ist es auch möglich mit dem Nagelspangenapplikator 1a breite Nagelspangen 23 (Breite 4 mm) auf einen Nagel 26 anzubringen.

Hierbei liegt die Nagelspange 23, zumindest teilweise, auf den Führungsschienen 9 auf, und die Orientierung der Nagelspange 23 wird durch die Führungsstifte 10 bestimmt.

Die sacklochartige Vertiefung 6a weist eine Magnetkammer 30 und eine Rückstellkammer 31 auf. Die Magnetkammer 30 hat eine Magnet-Ausdehnung, die in Grundkörper-Breitenrichtung y größer ist als eine Rückstell-Ausdehnung der Rückstellkammer 31. Entsprechend bildet sich beim Übergang von der Magnetkammer 30 zur Rückstellkammer 31 eine Kante 32 aus.

In der Rückstellkammer 31 ist eine Rückstelleinrichtung 33 angeordnet. Die Rückstelleinrichtung 33 ist im vorliegenden Ausführungsbeispiel als einstückig gegossenes Kunststoff-Bauteil ausgebildet und weist als Teilkomponenten drei Rückstellelemente 34 auf, die jeweils als Federringe ausgebildet sind.

Das Applikator-Magnetelement 25a ist innerhalb der sacklochartigen Vertiefung 6a beweglich angeordnet und insbesondere auch dementsprechend geführt. Wie durch den Doppelpfeil 40 angedeutet, lässt sich das Applikator-Magnetelement 25a in Grundkörper-Längsrichtung x des Nagelspangenapplikators 1a über eine Wegstrecke 41 hin- und herbewegen. Es ist außerdem in direktem physischen Kontakt mit der Rückstelleinrichtung 33. Das Applikator-Magnetelement 25a hat eine T-förmige Längsschnittfläche und weist einen Magnet-Breitenkörper 35 sowie einen Magnet-Längskörper 36 auf.

Der Magnet-Breitenkörper 35 ist dabei in direktem physischen Kontakt mit einem der Rückstellelemente 34. Der Magnet-Breitenkörper 35 ist vollständig in der Magnetkammer 30 angeordnet.

Die sacklochartige Vertiefung 6a weist zu ihrer der ersten Grundkörper-Stirnseite 3a zugewandten oberen Öffnung hin eine Verjüngung 37 auf. Beim Übergang von der Magnetkammer 30 zur Verjüngung 37 bildet sich eine Kante 38 aus.

Der Magnet-Längskörper 36 ist, zumindest teilweise, in der Verjüngung 37 angeordnet.

Die sacklochartige Vertiefung 6a inklusive der Magnetkammer 30 sowie der Rückstellkammer 31 hat außer der oberen Öffnung in einer der Grundkörper-Längsseiten 12 insbesondere auch eine (in der Schnittdarstellung gemäß Fig. 7 nicht gezeigte) seitliche Öffnung zum Einsetzen des Applikator-Magnetelements 25a und der Rückstelleinrichtung 33. Diese seitliche Öffnung ist durch einen insbesondere abnehmbaren Deckel verschlossen.

Fig. 7 zeigt den Nagelspangenapplikator 1a, und insbesondere das Applikator-Magnetelement 25a, in seiner Ruhelage, also ohne dass die Nagelspange 23 auf einem Nagel 26 angebracht wird. In diesem Fall weist das Applikator-Magnetelement 25a, insbesondere der Magnet-Längskörper 36 eine Deckfläche 39 auf, die parallel (und ggf. auch fluchtend) zu den beiden Anlageflächen 4a angeordnet ist. Die optionale fluchtende Anordnung ist bei dem Ausführungsbeispiel gemäß Fig. 7 nicht gegeben, da das Spangen-Magnetelement 24 als ausgedehnter Körper ausgeführt ist. Das Spangen-Magnetelement 24 kann aber bei einem nicht gezeigten alternativen Ausführungsbeispiel insbesondere auch als Magnetlack ausgebildet sein, der keine wesentliche Ausdehnung in Grundkörper-Längsrichtung x des Nagelspangenapplikators 1a aufweist, so dass auch das angesprochene Fluchten der Deckfläche 39 mit den beiden Auflageflächen 4a gegeben ist.

Bei der in Fig. 7 gezeigten Verwendung der schmalen Nagelspange 23 entsteht dann ein direkter physischer Kontakt zwischen dem Applikator-Magnetelement 25a und dem Spangen-Magnetelement 24. Bei der nicht gezeigten Verwendung einer breiten Nagelspange 23 ergibt sich insbesondere ein kleiner freier Zwischenraum zwischen dem Applikator-Magnetelement 25 und dem Spangen-Magnetelement 24. Der Abstand zwischen dem Applikator-Magnetelement 25 und dem Spangen-Magnetelement 24 beträgt dann insbesondere weniger als 0,1 mm, so dass ebenfalls eine sehr starke magnetische Anziehung gegeben ist.

Bei der Applikation der Nagelspange 23 wird die Nagelspange 23 in die konkave Anpressausnehmung 5a hineingedrückt, wodurch das Applikator-Magnetelement 25a gegen die Rückstellkraft der Rückstelleinrichtung 33 in Grundkörper-Längsrichtung x in die sacklochartige Vertiefung 6a hineingedrückt wird. Die Rückstelleinrichtung 33 wird durch diese Bewegung des Applikator-Magnetelements 25a betätigt, d.h. insbesondere (weiter) gespannt. Der untere Bewegungsendpunkt des Applikator-Magnetelements 25a ist erreicht, sobald der Magnet-Breitenkörper 35 an der Kante 32 anschlägt. Die Kante 32 verhindert insbesondere, dass die Rückstelleinrichtung 33 zu stark komprimiert und damit beschädigt wird, z.B. aufgrund einer zu weit in die sacklochartige Vertiefung 6a hineinreichenden Bewegung des Applikator-Magnetelements 25a.

Die Wegstrecke 41, um die sich der Magnet-Breitenkörper 35 innerhalb der Magnetkammer 30 zwischen der Kante 38 als dem oberen Anschlag und der Kante 32 als dem unteren Anschlag in Grundkörper-Längsrichtung x bewegen kann, legt den Umfang der Längsbeweglichkeit des Applikator-Magnetelements 25a insgesamt fest.

Zwischen dem tiefsten Punkt der konkaven Anpressausnehmung 5a und den Auflägeflächen 4a ist ein Abstand 42 gebildet. Dieser Abstand 42 und die Wegstrecke 41 können insbesondere gleich groß sein. Dann kann das Applikator-Magnetelement 25a soweit in die sacklochartige Vertiefung 6a hineingedrückt werden, dass die Deckfläche 39 des Magnet-Längskörpers 37 nicht mehr (auch nicht teilweise) in der konkaven Anpressausnehmung 5a angeordnet ist, sondern sich vollständig innerhalb der sacklochartigen Vertiefung 6a befindet.

Nachdem die Nagelspange 23 auf dem Nagel 26 angebracht und der Nagelspangenapplikator 1a wieder vom Nagel 26 entfernt worden ist, entfällt die Betätigung der Rückstelleinrichtung 33. Die vorher gespannte Rückstelleinrichtung 33 übt nun eine Rückstellkraft auf das Applikator-Magnetelement 25a aus, wodurch sich das Applikator-Magnetelement 25a zurück in seine obere Ruhelage bewegt. In dieser Ruhelage sind der Nagelspangenapplikator 1a und insbesondere das Applikator-Magnetelement 25a dazu bereit eine weitere Nagelspange 23 für eine weitere Applikation dieser Nagelspange 23 auf einen anderen Nagel 26 aufzunehmen und sicher zu halten.

## Patentansprüche

1. Nagelspangenapplikator zur Applikation einer mit einem Spangen-Magnetelement (24) versehenen Nagelspange (23) auf einem zu korrigierenden Nagel (26) einer Zehe (27) oder eines Fingers (27), aufweisend
a) einen länglichen Grundkörper (2; 2a), der sich in eine Grundkörper-Längsrichtung (x), eine Grundkörper-Breitenrichtung (y) und eine Grundkörper-Tiefenrichtung (z) erstreckt und eine senkrecht zu der Grundkörper-Längsrichtung (x) orientierte erste Grundkörper-Stirnseite (3; 3a) hat, die einen Applikationsbereich bildet, und
b) ein im Applikationsbereich angeordnetes Applikator-Magnetelement (25; 25a) zur Wechselwirkung mit dem Spangen-Magnetelement (24) und zur dadurch bedingten lösbaren Halterung der zu applizierenden Nagelspange (23) am Applikationsbereich, **dadurch gekennzeichnet, dass**
c) der Applikationsbereich zwei Auflageflächen (4; 4a) zur Auflage der zu applizierenden Nagelspange (23) und eine zwischen den beiden Auflageflächen (4; 4a) angeordnete konkave Anpress-Ausnehmung (5; 5a) aufweist.

2. Nagelspangenapplikator nach Anspruch 1, **dadurch gekennzeichnet, dass** die konkave Anpress-Ausnehmung (5; 5a) in Grundkörper-Breitenrichtung (y) mittig in der ersten Grundkörper-Stirnseite (3; 3a) angeordnet ist.

3. Nagelspangenapplikator nach Anspruch 1 oder 2, **dadurch gekennzeichnet, dass** an einem Boden der konkaven Anpress-Ausnehmung (5; 5a) eine sacklochartige Vertiefung (6; 6a) angeordnet ist, in die das Applikator-Magnetelement (25; 25a) zumindest teilweise eingesetzt ist.

4. Nagelspangenapplikator nach einem der vorhergehenden Ansprüche, **dadurch gekennzeichnet, dass** bei auf den beiden Auflageflächen (4; 4a) losem Aufliegen der Nagelspange (23) zwischen dem Spangen-Magnetelement (24) und dem Applikator-Magnetelement (25; 25a) ein freier Zwischenraum gegeben ist.

5. Nagelspangenapplikator nach einem der vorhergehenden Ansprüche, **dadurch gekennzeichnet, dass** die erste Grundkörper-Stirnseite (3; 3a) durch zwei Stirnseiten-Längsränder (7; 7a) und zwei Stirnseiten-Querränder (8) begrenzt ist und jede Auflagefläche (4) sich jeweils bis zu einem der Stirnseiten-Querränder (8) der ersten Grundkörper-Stirnseite (3; 3a) erstreckt.

6. Nagelspangenapplikator nach einem der vorhergehenden Ansprüche, **dadurch gekennzeichnet, dass** jede Auflagefläche (4; 4a) Führungselemente (9, 10) zur längsseitigen Führung der Nagelspange (23) aufweist.

7. Nagelspangenapplikator nach Anspruch 6, **dadurch gekennzeichnet, dass** die Führungselemente (9, 10) zur Führung der Nagelspange (23) längs der Grundkörper-Breitenrichtung (y) ausgestaltet sind.

8. Nagelspangenapplikator (1) nach einem der Ansprüche 6 oder 7, **dadurch gekennzeichnet, dass** die Führungselemente (9, 10) als Führungsstifte (10) oder als Führungsschienen (9) ausgebildet sind.

9. Nagelspangenapplikator (1) nach einem der Ansprüche 6 bis 8, **dadurch gekennzeichnet, dass** die Führungselemente (9, 10) als Kombinationselemente ausgestaltet sind, sodass an jeder Auflagefläche (4) in Grundkörper-Tiefenrichtung (z) gesehen zwei Aufnahmeabstände gebildet sind, wodurch zu applizierende Nagelspangen (23) mit zwei verschiedenen Spangenbreiten auflegbar sind.

10. Nagelspangenapplikator nach einem der vorhergehenden Ansprüche, **dadurch gekennzeichnet, dass** der Grundkörper (2; 2a) an einer der ersten Grundkörper-Stirnseite (3; 3a) gegenüberliegenden zweiten Grundkörper-Stirnseite (20) mit zwei voneinander beabstandeten Anpressspitzen (22) ausgestattet ist.

11. Nagelspangenapplikator nach einem der vorhergehenden Ansprüche, **dadurch gekennzeichnet, dass** der Grundkörper (2; 2a) zwei sich in die Grundkörper-Längsrichtung (x) erstreckende schmale Grundkörperlängsseiten (12) hat, die mit mindestens einer konkaven Messschablonen-Ausnehmung (13) zur Längenbestimmung der zu applizierenden Nagelspane (23) ausgestattet sind.

12. Nagelspangenapplikator nach Anspruch 11, **dadurch gekennzeichnet, dass** der Grundkörper (2; 2a) angrenzend an die mindestens eine Messschablonen-Ausnehmung (13) ein bezogen auf die mindestens eine Messschablonen-Ausnehmung (13) mittig angeordnetes Kennzeichnungs-Hilfsmittel (14) zur Kennzeichnung der Mitte auf dem zu korrigierenden Nagel (26) aufweist.

13. Nagelspangenapplikator nach einem der vorhergehenden Ansprüche, **dadurch gekennzeichnet, dass** der Grundkörper (2; 2a) am Applikationsbereich eine Mittenmarkierung (11) aufweist.

14. Nagelspangenapplikator nach einem der vorhergehenden Ansprüche, **gekennzeichnet durch** eine im Applikationsbereich angeordnete Rückstelleinrichtung (33), wobei das Applikator-Magnetelement (25a) insbesondere in Grundkörper-Längsrichtung (x) beweglich ist und an der Rückstelleinrichtung (33) angeordnet ist.

15. Nagelspangenapplikator nach Anspruch 14, **dadurch gekennzeichnet, dass** die Rückstelleinrichtung (33) mindestens ein Rückstellelement (34) aufweist.

## Claims

1. Nail brace applicator for applying a nail brace (23), provided with a brace magnet element (24), to a nail (26) to be corrected of a toe (27) or finger (27), comprising
a) an elongate base body (2; 2a), which extends in a base body length direction (x), a base body width direction (y) and a base body depth direction (z) and has a first base body end face (3; 3a), orientated perpendicular to the base body length direction (x) and forming an application region, and
b) an applicator magnet element (25; 25a) arranged in the application region, for interacting with the brace magnet element (24) and for thus releasably holding the nail brace (23) to be applied on the application region,
**characterised in that**
c) the application region has two support faces (4; 4a), for supporting the nail brace (23) to be applied, and a concave pressure recess (5; 5a), arranged between the two support faces (4; 4a).

2. Nail brace applicator according to claim 1, **characterised in that** the concave pressure recess (5; 5a) is arranged in the first base body end face (3; 3a) centrally in the base body width direction (y).

3. Nail brace applicator according to either claim 1 or claim 2, **characterised in that** a blind-hole depression (6; 6a), into which the applicator magnet element (25; 25a) is inserted at least in part, is arranged on a floor of the concave pressure recess (5; 5a).

4. Nail brace applicator according to any of the preceding claims, **characterised in that** when the nail brace (23) rests loosely on the two support faces (4; 4a) a free space is formed between the brace magnet element (24) and the applicator magnet element (25; 25a).

5. Nail brace applicator according to any of the preceding claims, **characterised in that** the first base body end face (3; 3a) is delimited by two end face longitudinal edges (7; 7a) and two end face transverse edges (8), and each support face (4) extends as far as one of the end face transverse edges (8) of the first base body end face (3; 3a).

6. Nail brace applicator according to any of the preceding claims, **characterised in that** each support face (4; 4a) has guide elements (9, 10) for longitudinally guiding the nail brace (23).

7. Nail brace applicator according to claim 6, **characterised in that** the guide elements (9, 10) are configured to guide the nail brace (23) along the base body width direction (y).

8. Nail brace applicator (1) according to either claim 6 or claim 7, **characterised in that** the guide elements (9, 10) are formed as guide pins (10) or as guide rails (9).

9. Nail brace applicator (1) according to any of claims 6 to 8, **characterised in that** the guide elements (9, 10) are configured as combination elements, in such a way that, on each support face (4), as seen in the base body depth direction (z), two receiving intervals are formed, making it possible to place nail braces (23) to be applied having two different brace widths.

10. Nail brace applicator according to any of the preceding claims, **characterised in that** the base body (2; 2a) is equipped with two pressure tips (22), spaced apart from one another, on a second base body end face (20) opposite the first base body end face (3; 3a).

11. Nail brace applicator according to any of the preceding claims, **characterised in that** the base body (2; 2a) has two narrow base body longitudinal faces (12), extending in the base body length direction (x) and equipped with at least one concave measuring template recess (13) for determining the length of the nail brace (23) to be applied.

12. Nail brace applicator according to claim 11, **characterised in that** the base body (2; 2a) has, adjacent to the at least one measuring template recess (13), an indicator (14), arranged centrally with respect to the at least one measuring template recess (13), for indicating the centre on the nail (26) to be corrected.

13. Nail brace applicator according to any of the preceding claims, **characterised in that** the base body (2; 2a) has a centre marking (11) on the application region.

14. Nail brace applicator according to any of the preceding claims, **characterised by** a return device (33) arranged in the application region, the applicator magnet element (25a) in particular being movable in the base body length direction (x) and being arranged on the return device (33).

15. Nail brace applicator according to claim 14, **characterised in that** the return device (33) has at least one return element (34).

## Revendications

1. Applicateur d'agrafe d'ongle destiné à l'application d'une agrafe d'ongle (23) pourvue d'un élément magnétique d'agrafe (24) sur un ongle (26) à corriger d'un orteil (27) ou d'un doigt (27), comprenant
a) un corps de base (2 ; 2a) allongé, qui s'étend dans une direction de la longueur du corps de base (x), une direction de la largeur du corps de base (y) et une direction de la profondeur du corps de base (z) et qui possède une première face frontale de corps de base (3 ; 3a) orientée perpendiculairement à la direction de la longueur du corps de base (x) et formant une zone d'application, et
b) un élément magnétique d'applicateur (25 ; 25a) disposé dans la zone d'application, destiné à l'interaction avec l'élément magnétique d'agrafe (24) et à la fixation détachable, inhérente à ladite interaction, de l'agrafe d'ongle (23) à appliquer sur la zone d'application,
**caractérisé en ce que**
c) la zone d'application comporte deux surfaces d'appui (4 ; 4a) destinées à l'appui de l'agrafe d'ongle (23) à appliquer et un évidement de pression (5 ; 5a) concave disposé entre les deux surfaces d'appui (4 ; 4a).

2. Applicateur d'agrafe d'ongle selon la revendication 1, **caractérisé en ce que** l'évidement de pression (5 ; 5a) concave est disposé dans la première face frontale de corps de base (3 ; 3a) de manière centrée dans la direction de la largeur du corps de base (y).

3. Applicateur d'agrafe d'ongle selon la revendication 1 ou 2, **caractérisé en ce que,** sur un fond de l'évidement de pression (5 ; 5a) concave, se trouve un creux (6 ; 6a) de type trou borgne, dans lequel l'élément magnétique d'applicateur (25 ; 25a) est au moins en partie inséré.

4. Applicateur d'agrafe d'ongle selon l'une des revendications précédentes, **caractérisé en ce que,** lorsque l'agrafe d'ongle (23) repose librement sur les deux surfaces d'appui (4 ; 4a), un espace intermédiaire libre est présent entre l'élément magnétique d'agrafe (24) et l'élément magnétique d'applicateur (25 ; 25a).

5. Applicateur d'agrafe d'ongle selon l'une des revendications précédentes, **caractérisé en ce que** la première face frontale de corps de base (3 ; 3a) est limitée par deux bords longitudinaux de face frontale (7 ; 7a) et deux bords transversaux de face frontale (8) et chaque surface d'appui (4) s'étend respectivement jusqu'à un des bords transversaux de face frontale (8) de la première face frontale de corps de base (3 ; 3a).

6. Applicateur d'agrafe d'ongle selon l'une des revendications précédentes, **caractérisé en ce que** chaque surface d'appui (4 ; 4a) comporte des éléments de guidage (9, 10) pour le guidage côté longitudinal de l'agrafe d'ongle (23).

7. Applicateur d'agrafe d'ongle selon la revendication 6, **caractérisé en ce que** les éléments de guidage (9, 10) sont réalisés pour le guidage de l'agrafe d'ongle (23) le long de la direction de la largeur du corps de base (y).

8. Applicateur d'agrafe d'ongle (1) selon l'une des revendications 6 et 7, **caractérisé en ce que** les éléments de guidage (9, 10) sont réalisés sous la forme de doigts de guidage (10) ou de rails de guidage (9).

9. Applicateur d'agrafe d'ongle (1) selon l'une des revendications 6 à 8, **caractérisé en ce que** les éléments de guidage (9, 10) sont réalisés sous la forme d'éléments de combinaison, de telle sorte que, sur chaque surface d'appui (4), vu dans la direction de la profondeur du corps de base (z), deux écarts de réception sont formés, moyennant quoi des agrafes d'ongle (23) à appliquer avec deux largeurs d'agrafe différentes peuvent être posées.

10. Applicateur d'agrafe d'ongle selon l'une des revendications précédentes, **caractérisé en ce que** le corps de base (2 ; 2a) est muni, sur une seconde face frontale de corps de base (20) opposée à la première face frontale de corps de base (3 ; 3a), de deux pointes de pression (22) espacées l'une de l'autre.

11. Applicateur d'agrafe d'ongle selon l'une des revendications précédentes, **caractérisé en ce que le** corps de base (2 ; 2a) possède deux côtés longitudinaux de corps de base (12) étroits s'étendant dans la direction longitudinale du corps de base (x), lesquels sont munis d'au moins un évidement de calibre de mesure (13) concave pour déterminer la longueur de l'agrafe d'ongle (23) à appliquer.

12. Applicateur d'agrafe d'ongle selon la revendication 11, **caractérisé en ce que** le corps de base (2 ; 2a) comporte, adjacente à l'au moins un évidement de calibre de mesure (13), une aide au marquage (14) disposée de manière centrée par rapport à l'au moins un évidement de calibre de mesure (13) pour marquer le centre sur l'ongle (26) à corriger.

13. Applicateur d'agrafe d'ongle selon l'une des revendications précédentes, **caractérisé en ce que** le corps de base (2 ; 2a) comporte, sur la zone d'application, un marquage du centre (11).

14. Applicateur d'agrafe d'ongle selon l'une des revendications précédentes, **caractérisé par** un dispositif de rappel (33) disposé dans la zone d'application, l'élément magnétique d'applicateur (25a) étant mobile en particulier dans la direction de la longueur du corps de base (x) et étant disposé sur le dispositif de rappel (33).

15. Applicateur d'agrafe d'ongle selon la revendication 14, **caractérisé en ce que** le dispositif de rappel (33) comporte au moins un élément de rappel (34).
